# FASCICULE DE BREVET EUROPEEN

(11) **EP 1 007 005 B1**
(45) Date de publication et mention de la délivrance du brevet: **15.09.2004**
(21) Numéro de dépôt: 98909235.8
(22) Date de dépôt: 13.03.1998
(51) Int. Cl.: A61K 9/20

(54) **COMPOSITIONS PHARMACEUTIQUES POUR LA LIBERATION CONTROLEE DE SUBSTANCES ACTIVES**
PHARMAZEUTISCHE ZUSAMMENSETZUNGEN ZUR KONTROLLIERTEN FREIGABE VON WIRKSTOFFEN
PHARMACEUTICAL COMPOSITIONS FOR CONTROLLED RELEASE OF ACTIVE SUBSTANCES

(30) Priorité: 14.03.1997 BE 9700225
(43) Date de publication de la demande: 14.06.2000
(73) Titulaire: UCB, S.A., 1070 Bruxelles (BE)
(72) Inventeur: FANARA, Domenico, B-4520 Wanze (BE); BERWAER, Monique, B-4350 Remincourt (BE); BOUQUELLE, Anne, B-7050 Jurbise (BE); DELEERS, Michel, B-1630 Linkebeek (BE)
(74) Mandataire: Lechien, Monique
(86) Numéro de dépôt international: PCT/BE1998/000033
(87) Numéro de publication internationale: WO 1998/041194

(56) Documents cités:
- EP-A- 0 396 404
- WO-A-94/06416
- WO-A-94/09761
- WO-A-95/34291

## Description

La présente invention concerne des compositions pharmaceutiques administrables par voie orale permettant la libération contrôlée de substances pharmaceutiquement actives ainsi que des méthodes de préparation de ces compositions pharmaceutiques.

Un des buts recherchés actuellement dans le développement de compositions pharmaceutiques administrables par voie orale est de contrôler la libération des substances pharmaceutiquement actives de manière à ce qu'elles puissent être administrées en peu de prises journalières, idéalement en une seule prise journalière.

Le contrôle de la libération de substances actives lors de l'administtation par voie orale peut se faire au moyen de compositions pharmaceutiques de type matriciel. Selon les excipients utilisés. on distingue trois types de matrices: les matrices inertes, hydrophiles et lipophiles. Par association d'excipients de ces différents types de matrices, on peut aussi créer des matrices mixtes.

Les matrices inertes comprennent des excipients appartenant essentiellement à la classe des polymères thermoplastiques. Ils sont inertes vis-à-vis des tissus biologiques, des autres excipients dans la formulation et de la substance active. Ils sont insolubles et non-digestibles dans les fluides du tractus gastro-intestinal. Parmi ceux-ci, on peut citer le chlorure de polvvinyle, le polyéthylène. les copolymères d'acétate et de chlorure de vinyle, les polyméthylméthacrylates, les polyamides, les silicones, l'éthylcellulose, le polystyrène ... Ils s'utilisent généralement à une concentration allant de 20 à 95%.

Les matrices hydrophiles comprennent des excipients gélifiants se répartissant en trois classes: les dérivés cellulosiques (hydroxypropylméthylcellulose, hydroxyéthylcellulose, hydroxypropylcellulose, méthylcellulose ...), les polysaccharides non cellulosiques (galactomannes, gomme guar, gomme caroube, gomme arabique, gomme sterculia, agar agar, alginates ...) et les polymères de l'acide acrylique (carbopols 934P et 974P ...). Ils s'utilisent généralement à une concentration de 20 à 70%.

Les matrices lipidiques comprennent des excipients gras de quatre types: les glycérides (mono- di- ou triglycérides: stéarine, palmitine, laurine, myristine, huiles de ricin ou de coton hydrogénées, précirol ...), les acides et les alcools gras (acides stéarique, palmitique. laurique: alcools stéarylique, cétylique, cétostéarylique ...), les esters d'acides gras (monostéarates de propylène glycol et de saccharose, distéarate de saccharose ...) et les cires (cire blanche, cire de cachalot ...). Ils s'utilisent généralement à une concentration de 10 à 50%.

La présence d'excipients de type matriciel dans des compositions pharmaceutiques permet dans bien des cas de ralentir la libération des substances actives par emprisonnement. Toutefois, ces excipients de type matriciel ne permettent pas toujours de ralentir suffisamment la libération de la substance active ou d'obtenir les profils de libération idéaux souhaités.

Par exemple, lorsque la composition pharmaceutique de type matriciel contient une substance qui doit impérativement être libérée dans l'estomac, la libération de la substance active pendant des durées suffisamment longues dépend non seulement du type d'excipients utilisés dans la composition, mais aussi du temps de résidence de la composition pharmaceutique dans l'estomac. C'est pourquoi plusieurs documents mentionnent l'utilisation de comprimés matriciels flottants.

En particulier, le brevet EP 205336 décrit des compositions pharmaceutiques pour la libération contrôlée de substances actives comprenant une matrice mixte obtenue à partir d'un mélange d'éthers de cellulose et d'un acide polyacrylique, de l'un de ses dérivés ou de leurs sels pharmaceutiquement acceptables, et comprenant en outre de 10 à 50% en poids, par rapport au poids total d'excipients matriciels, d'agent moussant effervescent. L'agent moussant effervescent permet de faire flotter la composition pharmaceutique dans le liquide gastrique, augmentant par là-même le temps de résidence dans l'estomac. L'agent moussant effervescent est un bicarbonate de métal alcalin ou alcalino-terreux utilisé de préférence en combinaison avec un acide organique.

Toutefois, la flottaison dans le liquide gastrique ne permet pas de résoudre d'autres problèmes observés dans le contexte du contrôle de la libération de substances actives à partir de compositions pharmaceutiques matricielles.

En effet, les quantités d'excipient matriciel nécessaires à une libération prolongée adéquate du principe actif peuvent se révéler trop importantes et rendre impossible ou trop onéreuse la réalisation de la forme pharmaceutique.

D'autre part, la libération de certaines substances actives dépend fortement du pH. Par exemple, certaines substances actives ne sont pas du tout libérées dans l'estomac, mais dans d'autres zones du tractus gastro-intestinal. En outre, pour une même zone du tractus gastro-intestinal, le profil de libération sera différent selon que l'administration de la composition a lieu simultanément ou non avec la prise d'aliments. Pour les substances actives dont la libération dépend du pH ambiant, il est donc souhaitable de trouver de nouvelles compositions matricielles permettant de régulariser la vitesse de libération de manière à ce que la substance active puisse être libérée à la même vitesse, quel que soit le pH du milieu.

Enfin, il est très courant que le profil de libération d'un principe actif à partir d'une forme matricielle soit irrégulier au cours du temps, c'est-à-dire que la cinétique de libération ne soit pas d'ordre zéro mais soit fonction de la racine carrée du temps. Une cinétique de libération d'ordre zéro correspond à une libération régulière et constante au cours du temps et est très recherchée pour garantir un effet thérapeutique régulier et de longue durée.

Parallèlement, il devient thérapeutiquement de plus en plus intéressant de pouvoir administrer simultanément par voie orale une substance active libérée immédiatement après administration, et la même ou une deuxième substance active libérée de manière progressive et régulière après administration. Dans le cas où la même substance active est simultanément administrée en libération immédiate et en libération prolongée, cela permet de libérer rapidement une dose suffisante de substance active pour déclencher l'effet désiré et de maintenir cet effet par une libération progressive et prolongée de la même substance active. Dans le cas où une substance active est libérée immédiatement et une autre substance active est libérée de manière prolongée, cela permet d'obtenir des effets thérapeutiques combinés au moyen de deux substances actives ayant des profils pharmacocinétiques très différents.

Dans ce contexte, la demande de brevet international WO94/09761 décrit une composition orale à libération retard comprenant :
a) un coeur matriciel constitué de :
   - sulfate de pseudoéphédrine
   - hydroxypropylméthylcellulose
   - éthylcellulose
   - phosphate dibasique de calcium
   - povidone
   - dioxyde de silicone
   - stéarate de magnésium
   et
b) un enrobage du coeur matriciel constitué de
   - loratadine
   - hydroxypropylméthylcellulose
   - polyéthylène glycol 400
   - polyéthylène glycol 3350.
La demande de brevet européen EP-A-0 396 404 décrit une composition orale à libération retard comprenant :
a) un coeur matriciel constitué de
   - ibuprofène
   - pseudoéphédrine
   - polymères hydrophiliques gélifiants, tels que hydroxypropylméthylcellulose
   - excipient tel que le phosphate dibasique de calcium
   - lubrifiant tel que stéarate de magnésium,
   et
b) un enrobage du coeur constitué de
   - loratadine
   - polymère hydrophilique
   - autres excipients.

Dans ce contexte, des compositions pharmaceutiques solides administrables par voie orale combinant dans une seule entité une partie à libération immédiate et une partie à libération retard ont été décrites. Cependant, ces compositions nécessitent des méthodes de préparation techniquement très sophistiquées et/ou ne permettent pas d'obtenir les profils de libération souhaités pour toutes les substances actives.

Nous venons maintenant de découvrir de manière surprenante de nouvelles compositions pharmaceutiques administrables par voie orale, permettant la libération contrôlée de substances pharmaceutiquement actives de manière telle qu'un effet thérapeutique satisfaisant est observé pendant des durées assez longues, par exemple en une, voire deux prises journalières seulement.

En particulier, les compositions selon la présente invention ne nécessitent pas des quantités excessives d'excipients matriciels et permettent la libération régulière et continue de substances actives pendant des périodes d'au moins 12 heures.

En outre, nous venons également de découvrir que ces nouvelles compositions pharmaceutiques à libération contrôlée peuvent être utilisées en combinaison avec une composition pharmaceutique à libération immédiate pour la même ou une autre substance active, dans une seule entité destinée à être administrée par voie orale.

La présente invention concerne donc des compositions pharmaceutiques administrables par voie orale, permettant la libération contrôlée d'au moins une substance active, comprenant
a) ladite au moins une substance active,
b) entre 5 et 60% en poids, par rapport au poids total de la composition, d'au moins un excipient, sélectionné parmi les matrices inertes, les matrices hydrophiles, les matrices lipidiques, les mélanges de matrices inertes et de matrices lipidiques, les mélanges de matrices hydrophiles et de matrices lipidiques, les mélanges de matrices hydrophiles et de matrices inertes, à l'exception des mélanges comprenant un acide polyacrylique et au moins une matrice hydrophile de type cellulosique;
c) entre 5 et 50% en poids, par rapport au poids total de la composition d'au moins un agent alcalinisant soluble dans une phase aqueuse dans des conditions de pH physiologique, sélectionné parmi les hydroxydes, les carbonates, les bicarbonates et les phosphates de métaux alcalins ou alcalino-terreux, le borate de sodium ainsi que les sels basiques d'acides organiques.

La présente invention est notamment illustrée par les Figures 1 à 5:
- Figure 1: Profils de biodisponibilité de la pseudoéphédrine obtenus avec des comprimés bicouche cétirizine (libération immédiate) / pseudoéphédrine (libération contrôlée);
- Figure 2: Cinétique de libération in vitro du trapidil; comprimés matriciels sans agent alcalinisant;
- Figure 3: Cinétique de libération in vitro du trapidil; comprimés matriciels avec agent alcalinisant;
- Figure 4: Cinétique de libération in vitro de l'hydrocodone; comprimés matriciels avec et sans agent alcalinisant;
- Figure 5: Profils de biodisponibilité du trapidil obtenus avec des comprimés matriciels pelliculés.

Les compositions pharmaceutiques selon la présente invention comprennent des excipients matriciels choisis parmi les matrices inertes, hydrophiles et lipophiles.

Des exemples de matrices inertes utilisables selon la présente invention sont: le chlorure de polyvinyle, le polyéthylène, les copolymères d'acétate et de chlorure de vinyle, les polyméthylméthacrylates, les polyamides, les silicones, l'éthylcellulose, le polystyrène ...

Des exemples de matrices hydrophiles utilisables selon la présente invention sont: les dérivés cellulosiques (hydroxypropylméthilcellulose, hydroxyéthylcellulose, hydroxypropylcellulose, méthylcellulose ...), les polysaccharides non cellulosiques (galactomannanes, gomme guar, gomme caroube, gomme arabique, gomme sterculia, agar agar, alginates ...) et les polymères de l'acide acrylique (carbopols 934P et 974P ...). Les matrices hydrophiles utilisées préférentiellement selon la présente invention sont les hydroxypropylcelluloses, telles que les METHOCEL K ou E. La teneur en excipients de type hydroxypropylméthylcellulose dans les compositions selon la présente invention est préférentiellement comprise entre 5 et 60% en poids par rapport au poids total de la composition.

Des exemples de matrices lipidiques utilisables selon la présente invention sont: les glycérides (mono- di- ou triglycérides: stéarine, palmitine, laurine, myristine, huiles de ricin ou de coton hydrogénées, précirol ..-), les acides et les alcools gras (acides stéarique, palmitique, laurique; alcools stéarylique, cétylique, cétostéarylique ...), les esters d'acides gras (monostéarates de propylène glycol et de saccharose, distéarate de saccharose ...)-et les cires (cire blanche, cire de cachalot ...).

Les excipients matriciels peuvent également se trouver sous la forme de mélange. Toutefois, les compositions pharmaceutiques de l'invention ne comprennent pas de mélanges comprenant un acide polyacrylique et au moins une matrice hydrophile de type cellulosique.

L'agent alcalinisant utilisable selon la présente invention doit être soluble dans une phase aqueuse dans des conditions de pH physiologique pour produire l'effet désiré. L'agent alcalinisant peut être choisi parmi les hydroxydes, les carbonates, les bicarbonates, les phosphates de métaux alcalins ou alcalino-terreux, le borate de sodium ainsi que des sels basiques d'acides organiques (exemple: citrate de sodium). Par contre les sels non solubles dans l'eau dans les conditions de pH physiologique tels que stéarate de magnésium, ou phosphate dibasique de calcium, ne conviennent pas selon la présente invention.

La quantité d'agent alcalinisant présente dans les compositions pharmaceutiques selon la présente invention est idéalement de 5 à 50% en poids par rapport au poids total de la composition.

En ce qui concerne les substances actives qui peuvent être présentes dans les compositions selon la présente invention, elles peuvent être de natures très variées.

Elles peuvent être choisies parmi les vasoconstricteurs, les antihistaminiques, les analgésiques, les antitussifs ... La demanderesse a notamment constaté que l'invention est particulièrement adaptée aux substances actives dont la base libre est moins soluble dans l'eau que ses sels pharmaceutiquement acceptables. Des exemples non limitatifs de telles substances actives sont la pseudoéphédrine, l'éphédrine, la phényléphrine, la phénylpropanolamine, le trapidil, l'hydrocodone, la cétirizine, l'éflétirizine, l'hydroxyzine, la méclozine, la buclizine, la pentoxyvérine, la codéine, la morphine, leurs isomères optiques ou leurs sels pharmaceutiquement acceptables.

Quant à la dose de substance active utilisée, elle dépend de la dose efficace et peut donc varier dans des limites très larges dépendant de ladite substance active.

Outre les composants précités, les compositions pharmaceutiques selon la présente invention peuvent également contenir d'autres excipients tels que des diluants (emcompress, lactose ...), des liants (avicel, amidons, polyvinylpyrrolidone ...), des désintégrants (amidons et amidons modifiés, dérivés cellulosiques, dérivés alginiques, pectines ...), des lubrifiants (talc, stéarate de magnésium, silice colloïdale ...), des agents de masquage de goût (α-cyclodextrine, β-cyclodextrine, γ-cyclodextrine et leurs dérivés alkylés), des arômes ou des colorants ainsi que des agents de pelliculage (exemple: dérivés cellulosiques, résines méthacryliques, chlorure de polyvinyle, nylons ...).

Les compositions pharmaceutiques selon la présente invention se présentent généralement sous une forme solide. Il est important de souligner que les effets bénéfiques de l'invention sont observés, quelle que soit la présentation de la forme galénique. Les compositions pharmaceutiques selon la présente peuvent se présenter sous la forme de comprimés, de granules, de microgranules, etc., ces formes étant enrobées ou non.

Les compositions pharmaceutiques à libération contrôlée selon la présente invention peuvent être préparées par les diverses méthodes conventionnelles connues de l'homme du métier.

Généralement, les compositions pharmaceutiques selon la présente invention sont préparées par un procédé comprenant les étapes successives suivantes:
i. préparation d'un mélange homogène contenant les composants a, b et c et les autres excipients éventuellement présents;
ii. la compression du mélange homogène obtenu à l'étape i, éventuellement après granulation.

La compression peut être de différents types, et se fait idéalement par compression directe. La granulation éventuelle à l'étape ii peut soit se faire par voie humide ou par voie sèche, ou encore par granulation par fusion (melt-granulation).

Selon un mode d'implémentation particulier de l'invention, les compositions pharmaceutiques à libération contrôlée selon l'invention sont utilisées en combinaison avec une ou plusieurs compositions pharmaceutiques permettant la libération immédiate de substances actives. Lorsque ces deux types de compositions sont présentes dans une même entité, cela permet d'obtenir en une seule administration à la fois la libération immédiate d'une première substance active et la libération prolongée de la même ou d'une deuxième substance active.

C'est pourquoi la présente invention concerne également des compositions pharmaceutiques administrables par voie orale comprenant
A. au moins une couche comprenant une substance active et des excipients qui permettent la libération immédiate de ladite substance active après administration, et
B. au moins une deuxième couche qui permet la libération contrôlée de la même ou d'une deuxième substance active, comprenant ladite même ou deuxième substance active, au moins un excipient de type matriciel, et au moins un agent alcalinisant.

En ce qui concerne la couche A, les excipients permettant la libération immédiate de la substance active peuvent être choisis parmi les diluants (emcompress, lactose ...), les liants (Avicel, amidons, polyvinylpyrrolidone ...), les désintégrants (amidons et amidons modifiés, dérivés cellulosiques, dérivés alginiques, pectines ...), les lubrifiants (talc, stéarate de magnésium, silice colloïdale ...), les agents de masquage de goût (α-cyclodextrine, β-cyclodextrine, γ-cyclodextrine et leurs dérivés alkylés), des arômes ou des colorants.

De telles compositions pharmaceutiques combinées peuvent être préparées selon diverses méthodes connues de l'homme du métier.

Plus particulièrement, ces compositions pharmaceutiques combinées peuvent se présenter sous la forme d'un comprimé dans lequel au moins une couche A est accolée à au moins une couche B. Dans ce cas, de telles compositions pharmaceutiques peuvent être préparées par un procédé comprenant les étapes successives suivantes:
1) préparation de mélanges homogènes séparés à partir des composants des couches A et B, et
2) compression des mélanges homogènes obtenus en 1) dans une comprimeuse multicouches.

Eventuellement, l'étape de compression 2) peut être précédée par une étape de granulation des mélanges homogènes obtenus à l'étape 1).

Les comprimeuses multicouches permettant de préparer ce genre de comprimés sont les comprimeuses multicouches de type Courtoy, Manesty, Hata, Fette, Killian, ...

Les comprimés multicouches sont particulièrement bien adaptés aux cas des associations de substances actives pour lesquelles des effets thérapeutiques bénéfiques bien particuliers ont été récemment observés, par exemple, pseudoéphédrine /cétirizine, hydrocodone/acétaminophen, hydrocodone libération immédiate/hydrocodone libération prolongée.

Les exemples qui suivent illustrent la présente invention, sans toutefois la limiter. Dans les exemples qui suivent, les pourcentages sont exprimés en poids par rapport au poids total des compositions.

### Exemple 1. Réduction de la quantité d'excipient matriciel.

Des comprimés A, B et C de pseudoéphédrine à libération contrôlée dosés à 120 mg ont été préparés par compression directe à partir de mélanges homogènes ayant respectivement les compositions présentées au Tableau 1.

Les comprimés A contiennent 16% d'excipient matriciel et 14,25% d'agent alcalinisant. Ils possèdent un poids moyen de 281,03 mg et une dureté de 104 N.

Les comprimés B et C, quant à eux, ne contiennent pas d'agent alcalinisant et contiennent respectivement 40% et 50% d'excipient matriciel. Ils possèdent respectivement un poids moyen de 298,3 mg et 402,25 mg, et leurs duretés respectives sont de 118 N et 137 N.

**Tableau 1 -**

| Composition des comprimés A, B et C | | | |
|---|---|---|---|
| constituants | mg/comprimé | | |
| | A | B | C |
| Pseudoéphédrine. HCl | 120 | 120 | 120 |
| Methocel K15M CR | 45 | 120 | - |
| Methocel K100M CR | - | - | 200 |
| Na₂CO₃ anhydre | 40 | - | - |
| Avicel pH 102 | 70,8 | 55,5 | 74 |
| Aérosil 200 | 1,4 | 1,5 | 2 |
| Stéarate de magnésium | 2,8 | 3 | 4 |

La cinétique de libération de la pseudoéphédrine à partir de ces 3 types de comprimés a été déterminée in vitro à l'aide de l'appareil de dissolution n° 1 de l'USP 23 (23^{ème} édition de la pharmacopée américaine). Les comprimés sont placés dans le panier qui subit 50 rotations par minute. Le milieu de dissolution est constitué de 500 ml d'eau distillée maintenue à 37°C. Chaque heure pendant 12 heures, un prélèvement est effectué dans le milieu de dissolution et la pseudoéphédrine est dosée par HPLC. Les résultats de ces dosages sont présentés dans le Tableau 2.

**Tableau 2 -**

| Pourcentages de libération de la pseudoéphédrine. | | | |
|---|---|---|---|
| Temps (h) | A | B | C |
| 0 | 0 | 0 | 0 |
| 1 | 31,87 | 41,56 | 38,81 |
| 2 | 46,04 | 57,89 | 51,69 |
| 3 | 56,4 | 71,4 | 62,43 |
| 4 | 65,07 | 81,43 | 72,02 |
| 5 | 75,41 | 86,34 | 77,65 |
| 6 | 75,95 | 90,44 | 83,53 |
| 7 | 77,7 | 93,01 | 85,21 |
| 8 | 79,6 | 94,16 | 88,48 |
| 10 | 82,82 | 96,21 | 92,16 |
| 12 | 85,36 | 94,05 | 93,07 |

Les résultats du Tableau 2 montrent que des profils de libération assez semblables sont observés dans les trois cas, la libération la plus lente étant observée pour les comprimés A. Ceci montre qu'un très bon contrôle de la libération peut être obtenu avec mains d'excipient matriciel lorsqu'un agent alcalinisant est présent dans la composition.

Les comprimés A, B et C ont également fait l'objet d'un essai de biodisponibilité chez l'homme sur une période de 32 heures. Dans cet essai, les profils de biodisponibilité obtenus avec les comprimés A, B et C ont été comparés au profil de biodisponibilité obtenu par l'administration de gélules à libération immédiate de pseudoéphédrine dosées à 60 mg administrées à 6 heures d'intervalle.

Huit sujets sains (hommes de 18 à 45 ans) ont participé à cette étude en cross-over, chaque sujet recevant les 4 formes avec une période de "wash-out" (rinçage) de 7 jours entre les deux administrations. Les sujets ont été soumis à des prélèvements sanguins pendant 32 heures selon les schémas suivants:
comprimés matriciels: 0; 0,5; 1; 1,5; 2; 3; 4; 5; 6; 7; 8; 10; 12; 16; 24 et 32 heures;
gélules: 0; 0,5; 1; 1,5; 2; 3; 4; 5; 6; 6,5; 7; 7,5; 8; 9; 10; 12; 16; 24 et 32 heures.
Les taux de pseudoèphédrine ont été déterminés par une méthode HPLC validée (détection UV).

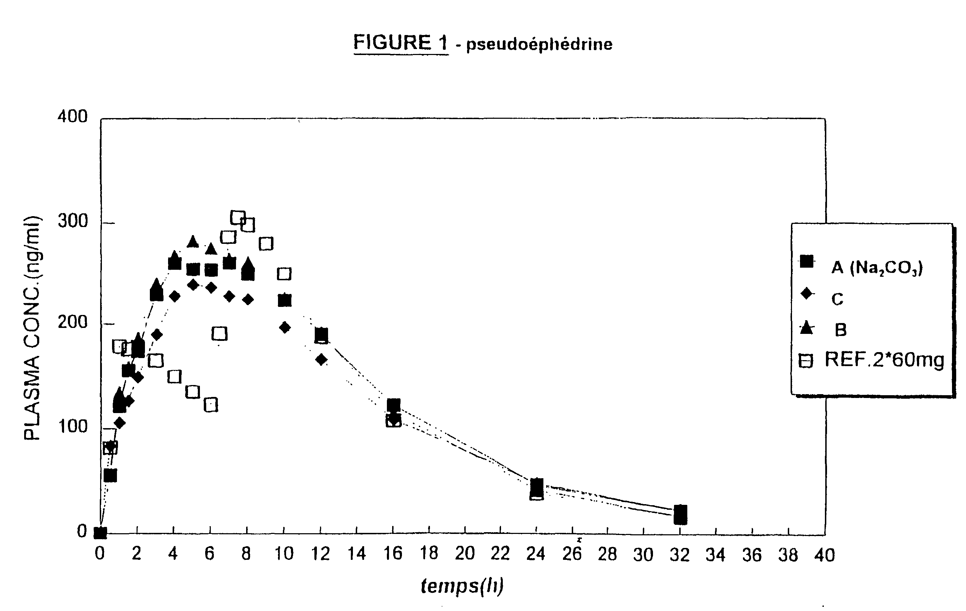

Les résultats de cette étude de biodisponibilité sont présentés à la Figure 1 et dans le Tableau 3, dans lequel SSC représente la surface sous la courbe, Cₘₐₓ représente la concentration maximale détectée et tₘₐₓ représente le temps nécessaire pour l'obtention de Cₘₐₓ.

**Tableau 3 -**

| Biodisponibilité de la pseudoéphédrine chez l'homme. | | | | |
|---|---|---|---|---|
| | Gélules (2x60 mg) | Comprimés (120 mg) | | |
| | | A | B | C |
| SSC (µg.h/ml) | 3672 | 4019 | 4028 | 3683 |
| Cₘₐₓ (µg/ml) | 391 | 276 | 295 | 259 |
| Tₘₐₓ (h) | 1,5 | 4,5 | 5,0 | 5,0 |

Les résultats de cette étude de biodisponibilité montrent que des profils de libération beaucoup plus réguliers sont obtenus avec des compositions pharmaceutiques comprenant des excipients matriciels dosées à 120 mg par rapport à celui obtenu avec deux administrations de comprimés à libération immédiate dosés à 60 mg.

D'autre part, la libération de la substance active se fait de manière comparable pour les comprimés A, B et C. Cet exemple illustre le fait que, pour obtenir une libération prolongée adéquate de la substance active, une quantité d'excipient matriciel beaucoup plus faible (16% au lieu de 40% ou 50%) est suffisante lorsque cet excipient est associé à un agent alcalinisant.

### Exemple 2. Effet du pelliculage.

Des comprimés de pseudoéphédrine à libération contrôlée dosés à 120 mg et contenant 15,4% de Methocel K15M CR et 13,7% de carbonate de sodium ont été préparés par compression directe à partir d'un mélange homogène ayant la composition présentée au Tableau 4 (comprimés D).

**Tableau 4 -**

| Comprimés D. | |
|---|---|
| constituants | mg/comprimé |
| Pseudoéphédrine . HCl | 120 |
| Methocel K15M CR | 45 |
| Na₂CO₃ anhydre | 40 |
| Avicel pH 102 | 82,5 |
| Aérosil 200 | 1,4 |
| Stéarate de magnésium | 2,8 |

Les comprimés D ont un poids moyen de 292,5 mg. Une partie de ces comprimés a fait l'objet d'un pelliculage à l'Opadry OY-B-28920 (alcool polyvinylique, lécithine, gomme xanthane, dioxyde de titane, talc). La cinétique de libération in vitro de la pseudoéphédrine à partir des comprimés D pelliculés ou non a été déterminée de la même manière qu'à l'exemple 1. Les résultats sont présentés au Tableau 5.

**Tableau 5 -**

| Pourcentages de libération de la pseudoéphédrine. | | |
|---|---|---|
| Temps (h) | non pelliculé | pelliculé |
| 0 | 0 | 0 |
| 1 | 33,7 | 30,4 |
| 2 | 47,5 | 45,6 |
| 3 | 57,9 | 56,5 |
| 4 | 65,8 | 65,7 |
| 5 | 71,9 | 72,0 |
| 6 | 77,1 | 76,8 |
| 7 | 80,7 | 80,2 |
| 8 | 83,4 | 83,1 |

Les résultats présentés au Tableau 5 montrent que le pelliculage n'affecte pas la cinétique de la libération de la substance active.

### Exemple 3. Effet de différents agents alcalinisants.

Des comprimés de pseudoéphédrine à libération contrôlée dosés à 180 mg et contenant 30% de Methocel K15M CR et 30% de carbonate de sodium (E), de bicarbonate de sodium (F) ou d'hydrogénophosphate de potassium (G) ont été préparés par compression directe à partir de mélanges homogènes ayant les compositions présentées au Tableau 6.

**Tableau 6 -**

| Comprimés E, F et G. | | | |
|---|---|---|---|
| constituants | mg/comprimé | | |
| | E | F | G |
| Pseudoéphédrine . HCl | 180 | 180 | 180 |
| Methocel K 15M CR | 180 | 180 | 180 |
| Na₂CO₃ anhydre | 180 | - | - |
| NaHCO₃ | - | 180 | - |
| K₂HPO₄ | - | - | 180 |
| Avicel pH 102 | 51 | 51 | 51 |
| Aérosil 200 | 3 | 3 | 3 |
| Stéarate de magnésium | 6 | 6 | 6 |

Les cinétiques de libération in vitro de la pseudoéphédrine pour les comprimés E, F et G, obtenues de la même manière que dans l'exemple 1 avec une rotation des paniers de 100 tpm, sont reprises dans le Tableau 7.

**Tableau 7 -**

| Pourcentages de libération de la pseudoéphédrine. | | | |
|---|---|---|---|
| Temps (h) | E | F | G |
| 0 | 0 | 0 | 0 |
| 1 | 17,1 | 29,2 | 28,9 |
| 2 | 23,3 | 45,6 | 45,2 |
| 3 | 28,9 | 57,7 | 56,2 |
| 4 | 34,5 | 67,3 | 65,1 |
| 5 | 39,1 | 74,9 | 73,3 |
| 6 | 44,1 | 81,6 | 79,8 |
| 7 | 49,2 | 86,1 | 85,2 |

Les résultats du Tableau 7 montrent que l'on obtient une libération prolongée avec les trois agents alcalinisants utilisés, l'effet étant plus marqué avec le carbonate de sodium.

### Exemple 4. Comprimés double couche pseudoéphédrine/cétirizine.

Des comprimés double couche dosés à 120 mg de pseudoéphédrine à libération contrôlée à 5 mg et de cétirizine à libération immédiate ont été préparés de la manière suivante (comprimés H).

Deux mélanges homogènes séparés (H_{A} et H_{B}) ont été préparés à partir des compositions présentées aux Tableaux 8 et 9.

Les mélanges H_{A} et H_{B} ont ensuite été comprimés dans une comprimeuse multicouche pour donner des comprimés double couche dans lesquels les couches sont accolées l'une à l'autre. Ces comprimés ont ensuite été pelliculés par l'Opadry OY-B-28920 (alcool polyvinylique, lécithine, gomme xanthane, dioxyde de titane, talc).

Dans ces comprimés, la couche à libération prolongée contient 16,1% d'excipient matriciel et 14,3% de carbonate de sodium.

**Tableau 8 -**

| Mélange H_{A}. | |
|---|---|
| composant | mg/comprimé |
| Cétirizine 2HCL | 5 |
| Tablettose | 41,3 |
| Avicel pH 102 | 22,65 |
| Aérosil 200 | 0,35 |
| Stéarate de magnésium | 0,7 |

**Tableau 9 -**

| Mélange H_{A}. | |
|---|---|
| composant | mg/comprimé |
| Pseudoéphédrine . HCl | 120 |
| Methocel K15M CR | 45 |
| Na₂CO₃ anhydre | 40 |
| Aérosil 200 | 1,4 |
| Stéarate de magnésium | 2,8 |

Les cinétiques de libération in vitro de la pseudoéphédrine et de la cétirizine pour les comprimés H, obtenues de la même manière que dans l'exemple 1 avec une rotation des paniers de 100 tpm, sont reprises dans le Tableau 10.

**Tableau 10 -**

| Pourcentages de libération de la pseudoéphédrine et de la cétirizine. | | |
|---|---|---|
| Temps (h) | Pseudoéphédrine | Cétirizine |
| 0 | 0 | 0 |
| 0,25 | 11,2 | 80,2 |
| 0,5 | 21,4 | 82,7 |
| 0,75 | 32,0 | 86,0 |
| 1 | 37,8 | 86,6 |
| 2 | 58,2 | 89,7 |
| 4 | 83,7 | 93,4 |
| 6 | 97,9 | 97,5 |
| 8 | 102,4 | 99,5 |
| 10 | 104,6 | 101,2 |
| 12 | 105,8 | 101,8 |

### Exemple 5. Effet du pH.

Des comprimés I et J de trapidil à libération contrôlée dosés à 300 mg ont été préparés par compression directe à partir de mélanges homogènes ayant les compositions reprises au Tableau 11.

Les comprimés I et J contiennent 33,3% d'excipient matriciel; les comprimés I ne contiennent pas d'agent alcalinisant et les comprimes J contiennent 12,5% d'agent alcalinisant.

**Tableau 11 -**

| Compositions des comprimés I et J. | | |
|---|---|---|
| constituants | mg/comprimé | |
| | I | J |
| Trapidil | 300 | 300 |
| Methocel K100M CR | 200 | 200 |
| Na₂CO₃ anhydre | - | 75 |
| Avicel pH 102 | 91 | 16 |
| Aérosil 200 | 3 | 3 |
| Stéarate de magnésium | 6 | 6 |

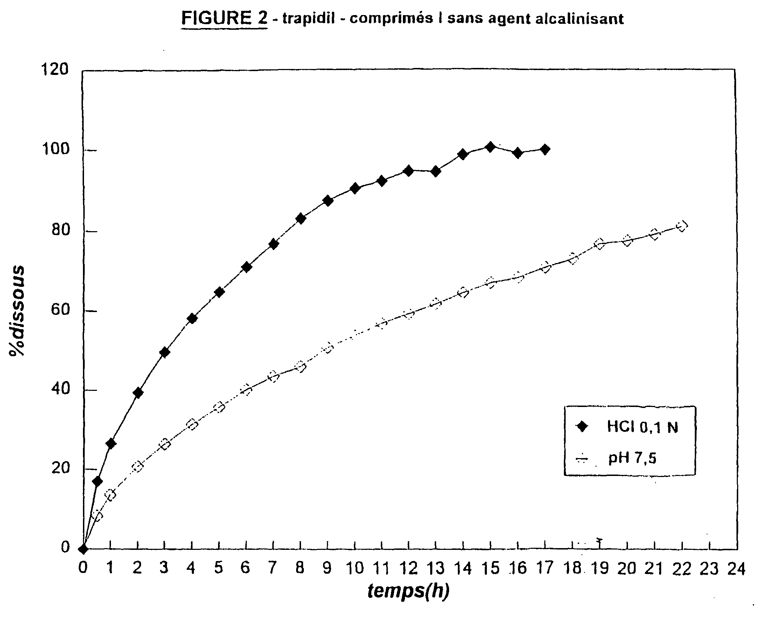

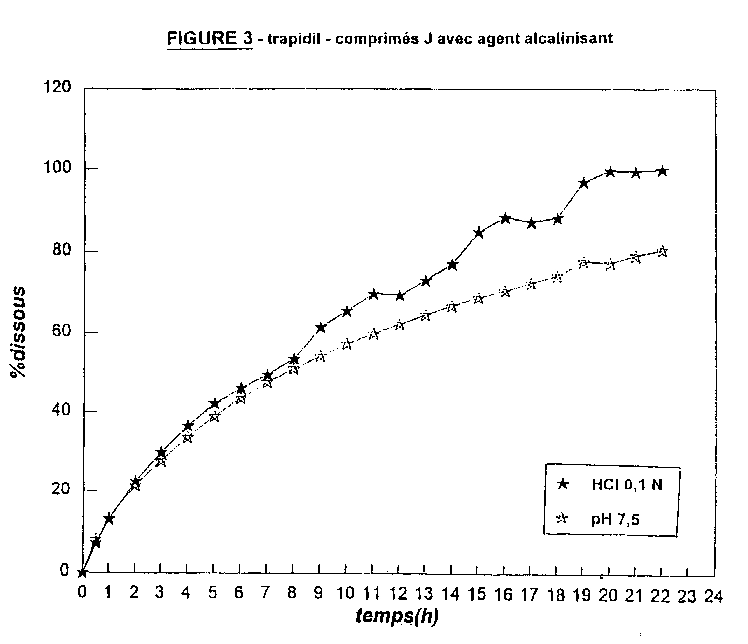

Les poids moyens et les duretés des comprimés I et J sont respectivement de 605,3 mg et 125 N (I) et de 597,7 mg et 79 N (J). Les cinétiques de libération in vitro du trapidil ont été effectuées selon la méthode décrite dans l'exemple 1 en utilisant deux milieux de dissolution: une solution 0,1 N d'acide chlorhydrique et une solution de tampon phosphate à pH 7,5. La vitesse de rotation des paniers était de 100 tpm. Les résultats de cette étude sont présentés au Tableau 12, ainsi qu'aux Figures 2 et 3.

**Tableau 12 -**

| Pourcentage de libération du trapidil en fonction du pH ambiant. | | | | |
|---|---|---|---|---|
| Temps (h) | HCl 0,1N | | Tampon pH 7.5 | |
| | I | J | I | J |
| 0 | 0 | 0 | 0 | 0 |
| 1 | 26,7 | 12,5 | 13,5 | 13,8 |
| 2 | 39,5 | 21,4 | 20,8 | 21,9 |
| 3 | 49,6 | 29,3 | 26,6 | 28,4 |
| 4 | 58,2 | 36,3 | 31,6 | 33,8 |
| 5 | 64,9 | 42,3 | 35,9 | 38,7 |
| 6 | 71,2 | 47,8 | 40,1 | 43,1 |
| 7 | 76,8 | 53,2 | 43,3 | 46,9 |
| 8 | 83,1 | 59,1 | 45,7 | 50,5 |
| 10 | 90,4 | 67,7 | 53,5 | 57,2 |
| 12 | 94,6 | 74,6 | 59,1 | 62,6 |
| 15 | 100,5 | 86,3 | 67,0 | 70,2 |
| 17 | 100,0 | 89,9 | 70,9 | 73,8 |

La Figure 2 et les résultats présentés au Tableau 12 pour les comprimés I montrent que ces comprimés présentent des cinétiques de libération très différentes en fonction du pH. Ces résultats montrent qu'une libération contrôlée et prolongée ne peut pas être obtenue en milieu fortement acide lorsqu'il n'y a pas d'agent alcalinisant dans la composition pharmaceutique.

La Figure 3 et les résultats présentés au Tableau 12 pour les comprimés J montrent qu'une libération prolongée de la substance active peut très bien être obtenue en milieu fortement acide lorsqu'un agent alcalinisant est présent dans la composition pharmaceutique.

### Exemple 6. Linéarisation de la cinétique de libération.

Des comprimés K et L d'hydrocodone à libération contrôlée dosés à 15 mg ont été préparés par compression directe à partir de mélanges homogènes ayant les compositions reprises au Tableau 13. Les comprimés K contiennent 56,7% d'excipient matriciel et ne contiennent pas d'agent alcalinisant; les comprimés L contiennent 43,3% d'excipient matriciel et 13,5% d'agent alcalinisant.

**Tableau 13 -**

| Compositions des comprimés K et L. | | |
|---|---|---|
| constituants | mg/comprimé | |
| | K | L |
| Bitartrate d'hydrocodone | 15 | 15 |
| Methocel K100M CR | 85 | 65 |
| Na₂CO₃ anhydre | - | 20 |
| Avicel pH 102 | 48 | 48 |
| Aérosil 200 | 0,5 | 0,5 |
| Stéarate de magnésium | 1,5 | 1,5 |

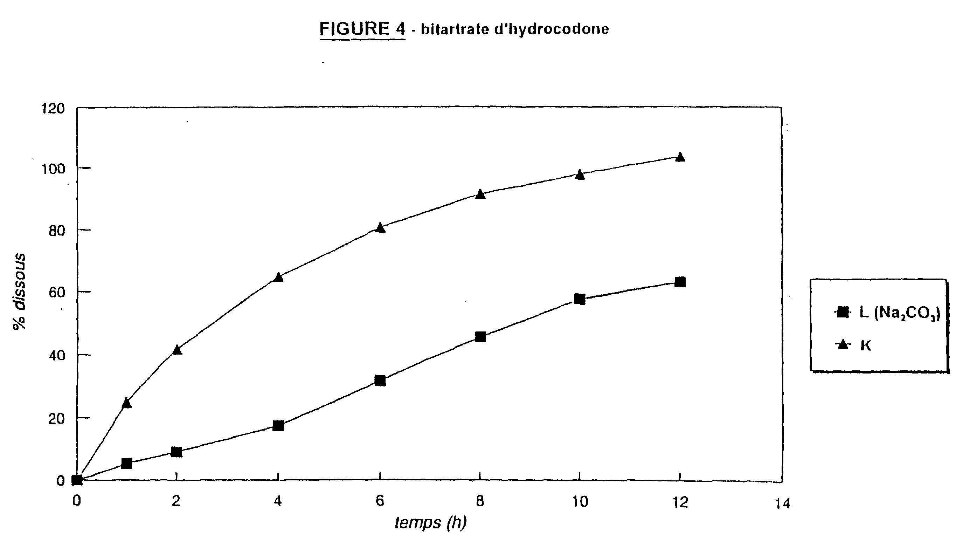

Les cinétiques de libération in vitro de l'hydrocodone à partir des comprimés K et L ont été déterminées à l'aide de l'appareil de dissolution n°2 de l'USP 23. Les comprimés sont placés dans le récipient de dissolution. La vitesse de rotation des pales est de 100 tours par minute. Le milieu de dissolution est constitué de 500 ml d'une solution de tampon phosphate à pH 5,8. Des prélèvements sont effectués pendant 12 heures dans le milieu de dissolution et l'hydrocodone est dosée par HPLC. Les résultats de cette étude sont présentés au Tableau 14 et à la Figure 4.

**Tableau 14 -**

| Pourcentages de libération de l'hydrocodone. | | |
|---|---|---|
| Temps (h) | K | L |
| 0 | 0 | 0 |
| 1 | 24,9 | 5,4 |
| 2 | 41,8 | 9,2 |
| 4 | 65,0 | 17,7 |
| 6 | 80,7 | 31,5 |
| 8 | 91,5 | 45,8 |
| 10 | 97,8 | 57,6 |
| 12 | 103,7 | 63,1 |

Les résultats présentés au Tableau 14 montrent que la présence d'agent alcalinisant dans la composition ralentit la libération de la substance active. En outre, comme le montre la Figure 4, la cinétique de libération est linéarisée, c'est-à-dire que la vitesse de libération reste constante au cours du temps.

Un autre test réalisé dans des conditions similaires et par dosage de l'hydrocodone par spectroscopie UV a montré également une vitesse de libération constante pendant 18 heures. De plus, la libération de la substance active était totale après 18 heures.

### Exemple 7. Comprimés double couche hydrocodone/hydrocodone.

Des comprimés double couche dosés à 15 mg d'hydrocodone constitués d'une couche à libération contrôlée dosée à 10 mg d'hydrocodone et une couche à libération immédiate dosée à 5 mg d'hydrocodone ont été préparés de la manière suivante (comprimés M).

Deux mélanges homogènes séparés (M_{A} et M_{B}) ont été préparés à partir des compositions présentées aux Tableaux 15 et 16.

Les mélanges M_{A} et M_{B} ont ensuite été comprimés dans une comprimeuse multicouche pour donner des comprimés double couche dans lesquels les deux couches sont accolées l'une à l'autre. Dans ces comprimés, la couche à libération prolongée contient 43,3% de Methocel K100M CR et 13,3% de carbonate de sodium.

**Tableau 15 -**

| Mélange M_{A}. | |
|---|---|
| constituants | mg/comprimé |
| Bitartrate d'hydrocodone | 5 |
| Tablettose | 28,2 |
| Avicel pH 102 | 16,1 |
| Aérosil 200 | 0,3 |
| Stéarate de magnésium | 0,4 |

**Tableau 16 -**

| Mélange M_{B}. | |
|---|---|
| constituants | mg/comprimé |
| Bitartrate d'hydrocodone | 10 |
| Methocel K100M CR | 43,3 |
| Na₂ CO₃ anhydre | 13,3 |
| Avicel pH 102 | 32,1 |
| Aérosil 200 | 0,3 |
| Stéarate de magnésium | 1 |

Les cinétiques de libération in vitro de l'hydrocodone pour les comprimés M ont été déterminées à l'aide de l'appareil de dissolution n°1 de l'USP 23. La vitesse de rotation des paniers était de 100 tours par minute. Le milieu de dissolution était constitué de 500 ml d'une solution de tampon phosphate à pH 5,8. Le dosage de l'hydrocodone a été effectué comme à l'exemple 6. Les résultats sont présentés au Tableau 17.

**Tableau 17 -**

| Pourcentages de libération de l'hydrocodone. | |
|---|---|
| Temps (h) | Hvdrocodone |
| 0 | 0 |
| 1 | 35,0 |
| 2 | 41,5 |
| 4 | 64,6 |
| 6 | 82,2 |
| 8 | 89,1 |
| 10 | 94,3 |
| 12 | 101,0 |

Les résultats présentés au Tableau 17 montrent que 35% d'hydrocodone ont déjà été libérés après 1 heure, ce qui correspond à la teneur en hydrocodone de la couche à libération immédiate (33,3% de la dose totale). Ensuite, la libération de l'hydrocodone se poursuit de manière progressive et régulière selon une cinétique comparable à celle observée dans l'exemple 6.

### Exemple 8. Comprimés d'hydrocodone - Matrice inerte

Des comprimés N et O d'hydrocodone à libération contrôlée dosés à 15 mg et contenant de l'acétate de cellulose en tant qu'excipient matriciel inerte ont été préparés par compression directe à partir de mélanges homogènes ayant les compositions reprises dans le Tableau 18. Les comprimés N contiennent 54 % d'excipient matriciel inerte et 13,3 % d'agent alcalinisant et les comprimés O contiennent 54 % d'excipient matriciel inerte et ne contiennent pas d'agent alcalinisant.

**Tableau 18 -**

| Compositions des comprimés N et O. | | |
|---|---|---|
| Constituants | mg/comprimé | |
| | N | O |
| Hydrocodone bitartrate | 15 | 15 |
| Acétate de cellulose CA398- 10NF | 81 | 81 |
| Emcompress | 32 | 52 |
| Carbonate de sodium | 20 | 0 |
| Aérosil 200 | 0,5 | 0,5 |
| Stéarate de magnésium | 1,5 | 1,5 |

La cinétique de libération de l'hydrocodone à partir de ces deux types de comprimés a été déterminée in vitro à l'aide de l'appareil de dissolution n° 1 de l'USP 23. Les comprimés sont placés dans le panier qui subit 100 rotations par minute. Le milieu de dissolution était constitué de 500 ml d'une solution de tampon phosphate à pH 5,8. Le dosage de l'hydrocodone a été effectué comme à l'exemple 6. Les résultats sont présentés au Tableau 19.

**Tableau 19 -**

| Pourcentages de libération de l'hydrocodone. | | |
|---|---|---|
| Temps (h) | Hydrocodone | |
| | N | O |
| 0 | 0 | 0 |
| 1 | 9,57 | 48,88 |
| 2 | 21,44 | 78,24 |
| 4 | 27,61 | 91,24 |
| 6 | 34,05 | 100,94 |
| 8 | 51,93 | 101,07 |
| 10 | 58,44 | 95,60 |
| 12 | 81,21 | 99,64 |

Les résultats présentés au Tableau 19 montrent que la présence d'agent alcalinisant dans la composition ralentit la libération de la substance active. En outre, comme dans l'exemple 6, la cinétique de libération est linéarisée.

### Exemple 9. Comprimés d'hydrocodone - matrice lipidique.

Des comprimés P et Q d'hydrocodone à libération contrôlée dosés à 15 mg et contenant de la CUTINA HR en tant qu'excipient matriciel lipidique ont été préparés par compression directe à partir de mélanges homogènes ayant les compositions reprises dans le Tableau 20. Les comprimés P contiennent 30,2 % d'excipient matriciel lipidique et 13,3 % d'agent alcalinisant et les comprimés Q contiennent 30,2 % d'excipient matriciel lipidique et ne contiennent pas d'agent alcalinisant.

**Tableau 20 -**

| Compositions des comprimés P et Q. | | |
|---|---|---|
| Constituants | mg/comprimé | |
| | P | O |
| Hydrocodone bitartrate | 15 | 15 |
| Cutina HR | 45,3 | 45,3 |
| Avicel PH 102 | 67,8 | 87,7 |
| Carbonate de sodium | 20 | 0 |
| Aérosil 200 | 0,5 | 0,5 |
| Stéarate de magnésium | 1,5 | 1,5 |

La cinétique de libération de l'hydrocodone à partir de ces deux types de comprimés a été déterminée in vitro à l'aide de l'appareil de dissolution n° 1 de l'USP 23. Les comprimés sont placés dans le panier qui subit 100 rotations par minute. Le milieu de dissolution était constitué de 500 ml d'une solution de tampon phosphate à pH 5,8. Le dosage de l'hydrocodone a été effectué comme à l'exemple 6. Les résultats sont présentés au Tableau 21.

**Tableau 21 -**

| Pourcentages de libération de l'hydrocodone. | | |
|---|---|---|
| Temps (h) | Hydrocodone | |
| | P | O |
| 0 | 0 | 0 |
| 1 | 5,91 | 58,76 |
| 2 | 12,50 | 84,46 |
| 3 | 23,05 | 91,96 |
| 4 | 29,01 | 91,47 |
| 6 | 52,07 | 101,84 |
| 8 | 68,96 | 102,63 |
| 10 | 80,92 | 103,37 |
| 12 | 90,73 | 104,92 |

Les résultats présentés au Tableau 21 montrent que la présence d'agent alcalinisant dans la composition ralentit la libération de la substance active. En outre, comme dans l'exemple 6, la cinétique de libération est linéarisée.

### Exemple 10. Comprimés d'hydrocodone - matrice mixte.

Des comprimés R et S d'hydrocodone à libération contrôlée dosés à 15 mg et contenant un mélange de METHOCEL K100MCR et d'EUDRAGIT RSPM en tant qu'excipients matriciels ont été préparés par compression directe à partir de mélanges homogènes ayant les compositions reprises dans le Tableau 20. Les comprimés R contiennent 43,3 % d'excipient matriciel et 13,3 % d'agent alcalinisant et les comprimés S contiennent 43,3 % d'excipient matriciel et ne contiennent pas d'agent alcalinisant.

**Tableau 22 -**

| Compositions des comprimés R et S. | | |
|---|---|---|
| Constituants | mg/comprimé | |
| | R | S |
| Hydrocodone bitartrate | 15 | 15 |
| Methocel K100MCR | 52,5 | 52,5 |
| Eudragit RSPM | 12,5 | 12,5 |
| Avicel PH 102 | 48 | 68 |
| Carbonate de sodium | 20 | 0 |
| Aérosil 200 | 0,5 | 0,5 |
| Stéarate de magnésium | 1,5 | 1,5 |

La cinétique de libération de l'hydrocodone à partir de ces deux types de comprimés a été déterminée in vitro à l'aide de l'appareil de dissolution n° 1 de l'USP 23. Les comprimés sont placés dans le panier qui subit 100 rotations par minute. Le milieu de dissolution était constitué de 500 ml d'une solution de tampon phosphate à pH 5,8. Le dosage de l'hydrocodone a été effectué comme à l'exemple 6. Les résultats sont présentés au Tableau 23.

**Tableau 23 -**

| Pourcentages de libération de l'hydrocodone. | | |
|---|---|---|
| Temps (h) | Hydrocodone | |
| | R | S |
| 0 | 0 | 0 |
| 1 | 9,10 | 26,05 |
| 2 | 18,12 | 42,80 |
| 3 | 27,81 | 49,76 |
| 4 | 38,13 | 59,32 |
| 6 | 63,41 | 70,16 |
| 8 | 81,76 | 78,13 |
| 10 | 88,12 | 83,22 |
| 12 | 101,69 | 88,73 |

Les résultats présentés au Tableau 23 montrent que la présence d'agent alcalinisant dans la composition ralentit la libération de la substance active. En outre, comme dans l'exemple 6, la cinétique de libération est linéarisée.

### Exemple 11. Comprimés matriciels pelliculés d'éflétirizine.

Des comprimés T, U et V d'éflétirizine à libération contrôlée dosés à 30 mg ont été préparés par granulation par voie humide du principe actif avec l'emcompress. Leur composition est reprise dans le Tableau 24.

**Tableau 24 -**

| Compositions des comprimés T, U et V. | | | |
|---|---|---|---|
| Constituants | mg/comprimé | | |
| | T | U | V |
| Eflétirizine.2HCl | 30 | 30 | 30 |
| Methocel K15MCR | 70 | 40 | 33,3 |
| Emcompress | 77 | 36,3 | 35 |
| Bicarbonate de sodium | 20 | 12 | 0 |
| Aérosil 200 | 1 | 0,7 | 0,7 |
| Stéarate de magnésium | 2 | 1 | 1 |
| Opadry Y1-7000 | 6 | 3,6 | 3 |

La cinétique de libération de l'éflétirizine à partir de ces trois types de comprimés a été déterminée in vitro à l'aide de l'appareil de dissolution n° 1 de l'USP 23. Les comprimés sont placés dans le panier qui subit 100 rotations par minute. Trois milieux de dissolution ont été testés: l'eau et des solutions tamponnées à pH 4,5 et 7,5. L'éflétirizine a été dosée par HPLC pour la solution tampon à pH 4,5 et par spectroscopie UV pour les autres solutions. Les résultats sont donnés dans le Tableau 25.

**Tableau 25 -**

| Pourcentages de libération de l'éflétirizine | | | | | | | | | |
|---|---|---|---|---|---|---|---|---|---|
| Temps (h) | Eflétirizine | | | | | | | | |
| | T | | | U | | | V | | |
| | pH 4,5 | Eau | pH 7,5 | pH 4,5 | Eau | pH 7,5 | pH 4,5 | Eau | pH 7,5 |
| 0 | 0 | 0 | 0 | 0 | 0 | 0 | 0 | 0 | 0 |
| 1 | 11,79 | 10,35 | 15,70: | 25,97 | 17,83 | 36,82 | 17,67 | 20,07 | 13,12 |
| 2 | 20,18 | 18,83 | 25,77: | 35,36 | 29,32 | 48,33 | 18,04 | 36,15 | 22,04 |
| 4 | 35,47 | 33,07 | 41,47 | 51,97 | 48,38 | 63,88 | 29,47 | 54,13 | 37,40 |
| 6 | 47,82 | 44,88 | 53,80 | 65,44 | 62,62 | 75,90 | 39,18 | 71,23 | 50,82 |
| 8 | 59,05 | 54,85 | 64,10 | 75,70 | 74,68 | 84,48 | 49,17 | 85,08 | 62,16 |
| 12 | 75,84 | 70,97 | 80,37 | 91,40 | 90,25 | 96,57 | 67,55 | 97,37 | 80,69 |
| 16 | 87,23 | 82,88 | 91,73 | 100,64 | 100,32 | 104,03 | 89,74 | 102,45 | 93,58 |
| 24 | 102,62 | 98,08 | 104,72 | 106,47 | 104,95 | 109,42 | 105,35 | 102,27 | 104,21 |

Les résultats présentés au Tableau 25 montrent que pour le comprimé V, qui ne contient pas d'alcalinisant, la cinétique de libération est fortement dépendante du pH. Pour les deux autres types de comprimés, cette différence est fortement réduite.

### Exemple 12. Comprimés matriciels pelliculés de trapidil.

Des matrices hydrophiles W, X et Y dosées à 300 mg de trapidil contenant un agent alcalinisant ont été préparées par granulation par voie humide du bicarbonate de sodium, du lactose et de la povidone; leur composition est reprise dans le Tableau 26.

**Tableau 26 -**

| Compositions des comprimés W, X et Y. | | | |
|---|---|---|---|
| Constituants | mg/comprimé | | |
| | W | X | Y |
| Trapidil | 300 | 300 | 300 |
| Methocel K15MCR | 0 | 0 | 200 |
| Methocel E4MCR | 150 | 0 | 0 |
| Natrosol 250 HHX | 0 | 150 | 0 |
| Bicarbonate de sodium | 60 | 60 | 60 |
| Avicel PH 101 | 50 | 100,8 | 0 |
| Lactose 100 mesh | 32 | 32 | 32 |
| Aérosil standard | 3 | 3,25 | 3 |
| Stéarate de magnésium | 3 | 1,95 | 3 |
| Povidone K30 | 2 | 2 | 2 |
| Opadry Y1-7000 | 18 | 20 | 18 |

Les comprimés W, X et Y ont fait l'objet d'un essai de biodisponibilité chez l'homme sur une période de 24 heures, chaque volontaire recevant 2 comprimés au temps O. Les profils de biodisponibilité obtenus avec les comprimés W, X et Y ont été comparés au profil obtenu par 3 administrations à 6 heures d'intervalle de gélules à libération immédiate de 200 mg (référence). Le profil après une administration unique d'une solution aqueuse contenant 200 mg de trapidil a également été déterminé.

Huit sujets sains (hommes de 18 à 45 ans) ont participé à cette étude en cross-over, chaque sujet recevant les 5 formes avec une période de "wash-out" (rinçage) de 7 jours entre deux administrations.

Les sujets ont été soumis à des prélèvements sanguins pendant 24 heures selon les schémas suivants:
- solution orale: 0; 0,33; 0,5; 0,67; 0,83; 1; 1,5; 2, 4 et 6 heures,
- gélule: 0; 0.33; 0,67; 1; 2; 4 et 6 heures après chacune des 3 administrations,
- comprimés matriciels: 0; 0,5; 1, 2; 4, 8; 12; 16; 20 et 24 heures.

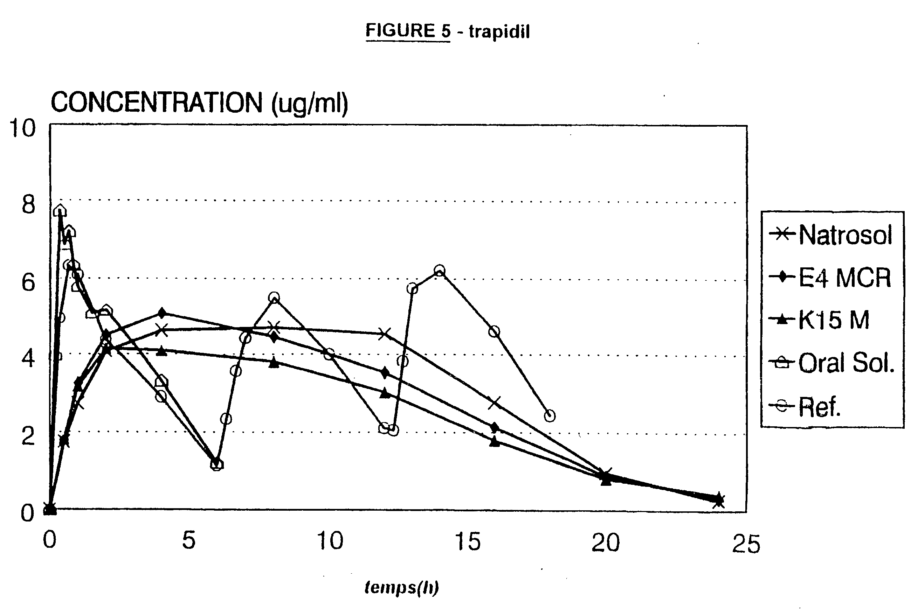

Les résultats de cette étude sont présentés à la Figure 5 et dans le tableau 27, dans lequel SSC représente la surface sous la courbe, Cₘₐₓ représente la concentration maximale détectée et tₘₐₓ représente le temps nécessaire pour l'obtention de Cₘₐₓ.

**Tableau 27 -**

| Biodisponibilité du trapidil chez l'homme. | | | | |
|---|---|---|---|---|
| | | Comprimés | | |
| | Gélules | W | X | Y |
| SSC₀₋₂₄ₕ (µg.h/ml) | 74,3 | 64,9 | 69,4 | 55,7 |
| Cₘₐₓ (µg/ml) | 7,55 | 5,05 | 5,07 | 4,34 |
| tₘₐₓ (h) | 1 | 4 | 10 | 3 |

Les résultats de cette étude montrent que les comprimés matriciels assurent une libération prolongée de la substance active. Par comparaison avec l'administration multiple de gélules à libération rapide, le comprimé X donne des résultats particulièrement intéressant avec un tₘₐₓ prolongé. Le Cₘₐₓ est diminué d'environ 30 % évitant ainsi les pics de concentration sanguine, mais présentant un plateau qui s'étend sur plus de 12 heures pour une absorption quasi équivalente de principe actif.

## Revendications

1. Composition pharmaceutique administrable par voie orale, permettant la libération contrôlée d'au moins une substance active, comprenant
a) ladite au moins une substance active,
b) entre 5 et 60% en poids, par rapport au poids total de la composition, d'au moins un excipient, sélectionné parmi les matrices inertes, les matrices hydrophiles, les matrices lipidiques, les mélanges de matrices inertes et de matrices lipidiques, les mélanges de matrices hydrophiles et de matrices inertes, à l'exception des mélanges comprenant un acide polyacrylique et au moins une matrice hydrophile de type cellulosique;
c) entre 5 et 50% en poids, par rapport au poids total de la composition d'au moins un agent alcalinisant soluble dans une phase aqueuse dans des conditions de pH physiologique, sélectionné parmi les hydroxydes, les carbonates, les bicarbonates et les phosphates de métaux alcalins ou alcalino-terreux, le borate de sodium ainsi que les sels basiques d'acides organiques.

2. Composition pharmaceutique selon la revendication 1, **caractérisée en ce que** la substance active est choisie parmi la pseudoéphédrine, l'éflétirizine, le trapidil et l'hydrocodone, leurs isomères optiques ou leurs sels pharmaceutiquement acceptables.

3. Composition pharmaceutique selon l'une quelconque des revendications 1 à 2, **caractérisée en ce que** l'excipient matriciel est de type hydroxypropylméthylcellulose.

4. Composition pharmaceutique selon l'une quelconque des revendications 1 à 3, **caractérisée en ce qu'**elle comprend en outre un ou plusieurs autres excipients pharmaceutiquement acceptable.

5. Composition pharmaceutique selon la revendication 3, **caractérisée en ce que** ledit un ou plusieurs autres excipients pharmaceutiquement acceptable est choisi parmi les diluants, les liants, les désintégrants, les lubrifiants, les agents de masquage de goût, les arômes, les colorants ou les agents de pelliculage.

6. Procédé de préparation d'une composition pharmaceutique selon l'une quelconque des revendications 1 à 5, **caractérisé en ce qu'**il comprend les étapes successives suivantes:
i. préparation d'un mélange homogène contenant les composants a, b et c et les autres excipients éventuellement présents;
ii. la compression du mélange homogène obtenu à l'étape i, éventuellement après granulation.

7. Composition pharmaceutique administrable par voie orale permettant la libération immédiate d'une première substance active et la libération prolongée de la même ou d'une deuxième substance active, comprenant
A. au moins une couche comprenant une substance active et des excipients qui permettent la libération immédiate de ladite substance active après administration, et
B. au moins une deuxième couche qui permet la libération contrôlée de la même ou d'une deuxième substance active, cette couche étant une composition pharmaceutique selon l'une quelconque des revendications 1 à 5.

8. Composition pharmaceutique selon la revendication 7, **caractérisée en ce que** la couche à libération immédiate A est accolée à la couche à libération prolongée B.

9. Procédé de préparation d'une composition pharmaceutique selon la revendication 7, **caractérisé en ce qu'**il comprend les étapes successives suivantes:
1) préparation de mélanges homogènes séparés à partir des composants des couches A et B, et;
2) compression des mélanges obtenus en 1) dans une comprimeuse multicouches.

10. Procédé de préparation selon la revendication 9, **caractérisé en ce que** l'étape de compression 2) est précédé par une étape de granulation des mélanges homogènes obtenus à l'étape 1).

## Patentansprüche

1. Oral verabreichbare pharmazeutische Zusammensetzung, die die kontrollierte Freisetzung wenigstens eines Wirkstoffs ermöglicht, umfassend
a) besagten wenigstens einen Wirkstoff,
b) zwischen 5 und 60 Gew.-%, bezogen auf das Gesamtgewicht der Zusammensetzung, wenigstens eines Hilfsstoffs, der ausgewählt ist unter den inerten Matrices, den hydrophilen Matrices, den Lipidmatrices, den Gemischen von inerten Matrices und Lipidmatrices, den Gemischen von hydrophilen Matrices und inerten Matrices, mit Ausnahme der Gemische, die eine Polyacrylsäure und wenigstens eine hydrophile Matrix vom Cellulosetyp umfassen,
c) zwischen 5 und 50 Gew.-%, bezogen auf das Gesamtgewicht der Zusammensetzung, wenigstens eines alkalisierenden Mittels, das in einer wässrigen Phase unter physiologischen pH-Bedingungen löslich ist, das ausgewählt ist unter den Hydroxiden, den Carbonaten, den Bicarbonaten und den Phosphaten von Alkalioder Erdalkalimetallen, Natriumborat sowie den basischen Salzen organischer Säuren.

2. Pharmazeutische Zusammensetzung gemäß Anspruch 1, **dadurch gekennzeichnet, dass** der Wirkstoff ausgewählt ist unter Pseudoephedrin, Efletirizin, Trapidil und Hydrocodon, ihren optischen Isomeren oder ihren pharmazeutisch annehmbaren Salzen.

3. Pharmazeutische Zusammensetzung gemäß einem der Ansprüche 1 bis 2, **dadurch gekennzeichnet, dass** der Matrixhilfsstoff vom Typ Hydroxypropylmethylcellulose ist.

4. Pharmazeutische Zusammensetzung gemäß einem der Ansprüche 1 bis 3, **dadurch gekennzeichnet, dass** sie außerdem einen oder mehrere weitere pharmazeutisch annehmbare Hilfsstoffe umfasst.

5. Pharmazeutische Zusammensetzung gemäß Anspruch 3, **dadurch gekennzeichnet, dass** besagte/r einer oder mehrere weitere pharmazeutisch annehmbare Hilfsstoffe ausgewählt sind unter den Verdünnungsmitteln, den Bindemitteln, den Sprengmitteln, den Gleitmitteln, den Geschmackmaskierungsmitteln, den Aromen, den Farbstoffen oder den Überzugsmitteln.

6. Verfahren zur Herstellung einer pharmazeutischen Zusammensetzung gemäß einem der Ansprüche 1 bis 5, **dadurch gekennzeichnet, dass** es die folgenden aufeinander folgenden Schritte umfasst:
i. Herstellung eines homogenen Gemischs, das die Bestandteile a, b und c und die anderen gegebenenfalls vorhandenen Hilfsstoffe umfasst;
ii. die Verpressung des im Schritt i erhaltenen homogenen Gemischs, gegebenenfalls nach Granulierung.

7. Oral verabreichbare pharmazeutische Zusammensetzung, die die sofortige Freisetzung eines ersten Wirkstoffs und die anhaltende Freisetzung des gleichen oder eines zweiten Wirkstoffs ermöglicht, umfassend
A. wenigstens eine Schicht, die einen Wirkstoff und Hilfsstoffe umfasst, die die sofortige Freisetzung besagten Wirkstoffs nach Verabreichung ermöglichen, und
B. wenigstens eine zweite Schicht, die die kontrollierte Freisetzung des gleichen oder eines zweiten Wirkstoffs ermöglicht, wobei diese Schicht eine pharmazeutische Zusammensetzung gemäß einem der Ansprüche 1 bis 5 ist.

8. Pharmazeutische Zusammensetzung gemäß Anspruch 7, **dadurch gekennzeichnet, dass** die Schicht mit sofortiger Freisetzung A mit der Schicht mit anhaltender Freisetzung B verbunden ist.

9. Verfahren zur Herstellung einer pharmazeutischen Zusammensetzung gemäß Anspruch 7, **dadurch gekennzeichnet, dass** es die folgenden aufeinander folgenden Schritte umfasst:
1) Herstellung von getrennten homogenen Gemischen aus den Bestandteilen der Schichten A und B und
2) Verpressung der in 1) erhaltenen Gemische in einer Mehrschichtpresse.

10. Verfahren zur Herstellung gemäß Anspruch 9, **dadurch gekennzeichnet, dass** dem Schritt zur Verpressung 2) ein Schritt zur Granulierung der im Schritt 1) erhaltenen homogenen Gemische vorausgeht.

## Claims

1. Orally administrable pharmaceutical composition permitting the controlled release of at least one active substance, comprising
a) said at least one active substance,
b) from 5 to 60 wt.%, based on the total weight of the composition, of at least one excipient selected from inert matrices, hydrophilic matrices, lipidic matrices, mixtures of inert matrices and lipidic matrices, mixtures of hydrophilic matrices and inert matrices, with the exception of mixtures comprising a polyacrylic acid and at least one hydrophilic matrix of the cellulose type;
c) from 5 to 50 wt.%, based on the total weight of the composition, of at least one alkalinising agent that is soluble in an aqueous phase under physiological pH conditions, selected from alkali metal and alkaline earth metal hydroxides, carbonates, bicarbonates and phosphates, sodium borate and also the basic salts of organic acids.

2. Pharmaceutical composition according to claim 1, **characterised in that** the active substance is selected from pseudoephedrine, efletirizine, trapidil and hydrocodone, their optical isomers and their pharmaceutically acceptable salts.

3. Pharmaceutical composition according to either claim 1 or claim 2, **characterised in that** the matrix excipient is of the hydroxypropylmethylcellulose type.

4. Pharmaceutical composition according to any one of claims 1 to 3, **characterised in that** it further comprises one or more other pharmaceutically acceptable excipients.

5. Pharmaceutical composition according to claim 3, **characterised in that** said one or more other pharmaceutically acceptable excipients are selected from diluents, binders, disintegrators, lubricants, taste-masking agents, flavourings, colourings and film-coating agents.

6. Process for the preparation of a pharmaceutical composition according to any one of claims 1 to 5, **characterised in that** it comprises the following successive steps:
i. preparation of a homogeneous mixture comprising components a, b and c and the other excipients optionally present;
ii. compression of the homogeneous mixture obtained in step i, optionally after granulation.

7. Orally administrable pharmaceutical composition permitting the immediate release of a first active substance and the sustained release of the same or of a second active substance, comprising
A. at least one layer comprising an active substance and excipients which permit the immediate release of said active substance following administration, and
B. at least one second layer which permits the controlled release of the same or of a second active substance, this layer being a pharmaceutical composition according to any one of claims 1 to 5.

8. Pharmaceutical composition according to claim 7, **characterised in that** the immediate-release layer A is coupled with the sustained-release layer B.

9. Process for the preparation of a pharmaceutical composition according to claim 7, **characterised in that** it comprises the following successive steps:
1) preparation of separate homogeneous mixtures from the components of layers A and B, and
2) compression of the mixtures obtained in 1) in a multi-layer compressing machine.

10. Preparation process according to claim 9, **characterised in that** the compression step 2) is preceded by a step in which the homogeneous mixtures obtained in step 1) are granulated.
